# DEMANDE DE BREVET EUROPEEN

(11) **EP 0 554 636 A1**
(43) Date de publication de la demande: **11.08.1993**
(21) Numéro de dépôt: 92403590.0
(22) Date de dépôt: 30.12.1992
(51) Int. Cl.: C07C 51/377, C07C 59/52, C07C 59/64, C07D 333/24, C07D 317/60

(54) **Procédé de préparation d'acides arylacétiques et de leurs sels de métaux alcalins**

(30) Priorité: 04.02.1992 FR 9201215
(71) Demandeur: SOCIETE FRANCAISE HOECHST Société anonyme dite:, F-92800 Puteaux (FR)
(72) Inventeur: Vallejos , Jean-Claude, F-75018 Paris (FR); Christidis, Yani, F-75019 Paris (FR)
(74) Mandataire: Rinuy, Santarelli

(57) **Abrégé**

Procédé d'obtention d'un acide arylacétique de formule générale (I)

**Ar - CHR - COOH (I)**

dans laquelle R = H, alc C₁-C₄, Ar = thiényle-2, thiényle-3, naphtyle-1, naphtyle-2, (méthoxy-2 naphtyle)-1, méthylènedioxy-3,4 phényle ou phényle de formule générale (II) 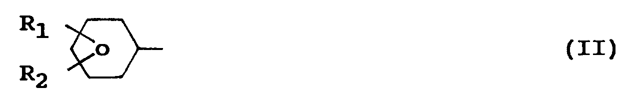
dans laquelle R₁ = H, OH ou alcoxy C₁-C₄, R₂ = H, alc C₁-C₄, alcoxy C₁-C₄ ou OH ainsi que leurs sels de métal alcalin, par réaction dans un solvant de l'acide arylglycolique correspondant de formule générale (III)

**Ar - CR(OH) - COOH (III)**

ou l'un des ses sels de métal alcalin avec un agent donneur d'hydrogène choisi dans le groupe constitué par : l'acide formique, ou l'un de ses sels de métal alcalin, l'acide phosphonique, l'acide phosphinique, ou l'un de leurs sels de métal alcalin, en présence d'un catalyseur de transfert d'hydrogène, que si désiré l'on isole ou l'on salifie, et application à la préparation de certains acides de formule (I).

## Description

La présente invention concerne un procédé de préparation d'acides arylacétiques et de leurs sels de métaux alcalins.

Les acides arylacétiques accessibles par le procédé de la présente invention sont les acides de formule générale (I)

**Ar - CHR - COOH (I)**

Dans laquelle R représente un atome d'hydrogène ou un radical alcoyle en C₁-C₄ et Ar représente un radical à caractère aromatique choisi parmi les radicaux suivants : thiényle-2, thiényle-3, naphtyle-1, naphtyle-2, (méthoxy-2 naphtyle)-1, méthylènedioxy-3,4 phényle, les phényles de formule générale (II) 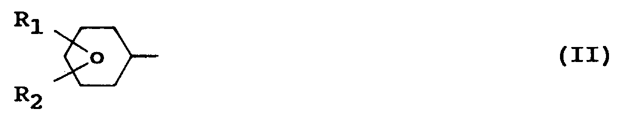
dans laquelle R₁ représente un atome d'hydrogène ou un radical hydroxyle ou alcoxyle en C₁-C₄ et R₂ représente un atome d'hydrogène ou un radical alcoyle en C₁-C₄ , alcoxyle en C₁-C₄ ou hydroxyle ainsi que leurs sels de métaux alcalins.

Le terme alcoyle en C₁-C₄ peut désigner, par exemple, un radical méthyle, éthyle, propyle, butyle, isopropyle, isobutyle, sec-butyle, tert-butyle.

Le terme alcoxyle en C₁-C₄ peut désigner, par exemple, un radical méthoxyle, éthoxyle, propoxyle, butoxyle, isopropoxyle, isobutoxyle, sec-butoxyle, tert-butoxyle.

Les acides arylacétiques de formule générale (I) et leurs sels de métaux alcalins sont largement décrits dans la littérature, et ce sont des matières premières intéressantes pour accéder à certaines substances présentant une activité thérapeutique, par exemple anti inflammatoire. Il est donc d'un grand intérêt de pouvoir les préparer d'une manière aussi rationnelle que possible. Il est connu notamment, de pouvoir accéder aux acides arylacétiques de formule générale (I) par hydrogènolyse, soit chimique, soit catalytique des acides arylglycoliques correspondants de formule générale (III)

**Ar - CR(OH) - COOH (III)**

dans laquelle Ar et R conservent la signification donnée précédemment (cf demandes de brevets européens N° 3825, 28375, 32374, 124407, 221815, 224401, 265793). Ces procédés exigent toutefois soit des matières premières rarement disponibles sur le marché, soit des réactifs coûteux.

Or la demanderesse a découvert avec étonnement qu'il était possible d'obtenir économiquement, et avec de bons rendements, un acide arylacétique de formule générale (I) ou son sel de métal alcalin en faisant réagir, au sein d'un solvant convenable, l'acide arylglycolique de formule générale (III) correspondant ou l'un de ses sels de métal alcalin, avec un agent donneur d'hydrogène convenable en présence d'un catalyseur de transfert d'hydrogène.

Le terme métal alcalin peut désigner, par exemple et de préférence, le sodium ou le potassium.

Des solvants utilisables sont, par exemple, l'eau, l'acide acétique, les mélanges eau-acide acétique en proportions variables, les solutions aqueuses diluées d'hydroxyde de sodium ou d'hydroxyde de potassium.

Des agents donneurs d'hydrogène convenables sont, par exemple, l'acide formique, ou l'un de ses sels de métal alcalin, l'acide phosphonique, l'acide phosphinique, ou l'un de leurs sels de métal alcalin.

Des catalyseurs de transfert d'hydrogène sont, par exemple, le palladium, le platine, le rhodium, éventuellement déposés sur un support solide amorphe tel que le charbon, le carbonate de calcium, le sulfate de baryum. De préférence, le catalyseur de transfert d'hydrogène est du palladium notamment déposé sur du charbon.

Le procédé selon la présente invention est avantageusement mis en oeuvre dans de l'eau ou dans un mélange eau-acide acétique. La réaction est menée de préférence à la pression atmosphérique, à une température supérieure à 50°C, préférentiellement supérieure ou égale à 80°C, en présence d'un excès d'agent de transfert d'hydrogène par rapport à la stoechiométrie, de préférence avec un excès de 10 à 50 % molaire.

En fin de réaction, l'acide arylacétique cherché, de formule générale (I) est très facilement isolé du milieu réactionnel par des moyens connus en soi. Avantageusement, après refroidissement à la température ambiante, le milieu réactionnel est filtré pour récupérer le catalyseur, puis le filtrat est concentré sous pression réduite pour éliminer les solvants, et l'huile résiduelle est dissoute dans de l'eau à un pH supérieur à 7 en présence d'un agent minéral alcalin tel que l'hydrogénocarbonate de sodium ou l'hydroxyde de sodium. La solution aqueuse, acidifiée ensuite à un pH=1 avec de l'acide chlorhydrique concentré, laisse déposer, généralement à l'état cristallisé, l'acide arylacétique cherché qui peut être, si nécessaire, purifié ultérieurement selon des moyens connus en soi, tels que la recristallisation par chaud et froid dans un solvant approprié.

Si désiré, l'acide peut être salifié en le sel de métal alcalin souhaité selon les méthodes usuelles, par exemple par action de l'hydroxyde de métal alcalin correspondant.

Le procédé de la présente invention ci-dessus décrit est avantageusement réalisé en dissolvant dans un mélange eau-acide acétique, une mole de sel de sodium de l'acide arylglycolique choisi de formule générale (III), en introduisant ensuite dans cette solution successivement du noir palladié à 4 ± 1 % de palladium, puis un excès de l'agent donneur d'hydrogène choisi, en chauffant le milieu réactionnel à une température supérieure à 80°C, jusqu'à transformation complète du produit de départ, laquelle est suivie par analyse chromatographique d'une prise d'essai prélevée régulièrement dans le milieu réactionnel, en éliminant ensuite le catalyseur par filtration et les solvants réactionnels par distillation sous pression réduite, en dissolvant l'huile résiduelle dans de l'eau à un pH supérieur à 7, et enfin, en précipitant de cette solution aqueuse l'acide recherché, par acidification à pH=1.

Selon une variante du procédé selon la présente invention, on opère en présence de quantités catalytiques d'ions chlorure. Avantageusement, ces ions chlorure sont apportés en dissolvant dans le milieu réactionnel du chlorure de sodium. Selon un mode avantageux, on réalise le procédé de la présente invention en présence de 0,1 à 0,01 mole de chlorure de sodium par mole d'acide arylglycolique de formule générale (III) mise en oeuvre.

Parmi les acides arylacétiques de formule générale (I) accessibles par le procédé de la présente invention, on peut citer :
- l'acide parahydroxyphénylacétique
- l'acide orthohydroxyphénylacétique
- l'acide paraméthoxyphénylacétique
- l'acide parahydroxyphényl-2 propanoïque.

C'est pourquoi la présente demande a aussi pour objet l'application du procédé ci-dessus décrit à l'obtention des acides arylacétiques de formule (I) précitée.
Les exemples suivants illustrent la présente invention.

### EXEMPLE 1

On chauffe 10 heures à l'ébullition, sous agitation, un mélange de 52 g (250 mmoles) de parahydroxymandélate de sodium cristallisé avec un molécule d'eau, 80 g d'acide acétique, 25 g d'eau, 12,9 g (280 mmoles) d'acide formique, 9 g (154 mmoles) de chlorure de sodium, et 1,56 g de noir palladié à 5 % en poids de palladium. La suspension, refroidie à la température ambiante, est ensuite filtrée, puis le filtrat incolore est concentré sous pression réduite. L'huile résiduelle est dissoute dans une solution aqueuse d'hydrogénocarbonate de sodium, puis la solution obtenue est acidifiée à pH=1, avec de l'acide chlorhydrique concentré. L'acide cherché cristallise spontanément, on le filtre, on le lave ensuite à l'eau, et on le sèche à poids constant sous pression réduite à 60°C. On obtient ainsi 35,75 g (235 mmoles) d'acide parahydroxyphénylacétique cristallisé pur, présentant un point de fusion de 148 ± 2°C (littérature F = 149-151°C, Beil., 10, 190). Le rendement est de 94 % de la théorie calculée par rapport à l'acide de départ.

### EXEMPLE 2

On chauffe 8 heures à l'ébullition, sous agitation, un mélange de 25,5 g (125 mmoles) de paraméthoxymandélate de sodium, 80 g d'acide acétique, 12,8 g (156 mmoles) d'acide phosphonique, 500 mg de chlorure de sodium et 255 mg de noir palladié à 5 % en poids de palladium. L'analyse d'une prise d'essai par chromatographie liquide sous haute pression ne révèle plus à ce stade de la réaction, de produit de départ.
La suspension refroidie à la température ambiante, est alors filtrée, puis le filtrat incolore est concentré sous pression réduite. L'huile résiduelle est reprise avec une solution aqueuse d'hydrogènocarbonate de sodium puis la solution obtenue est acidifiée à pH=1 avec de l'acide chlorhydrique concentré. L'acide paraméthoxyphénylacétique cherché cristallise spontanément, on le filtre, puis on le lave à l'eau par empatage, et enfin on le sèche à poids constant sous pression réduite à 60°C. On isole ainsi 20 g (120 mmoles) d'acide paraméthoxyphénylacétique cristallisé pur présentant un point de fusion de 84-85°C.

### EXEMPLE 3

A partir de l'orthohydroxymandélate de sodium on a préparé l'acide orthohydroxyphénylacétique de manière analogue à celle décrite aux exemples 1 et 2.

## Revendications

1. Procédé d'obtention d'un acide arylacétique de formule générale (I)
**Ar - CHR - COOH (I)**
dans laquelle R représente un atome d'hydrogène ou un radical alcoyle en C₁-C₄ et Ar représente un radical à caractère aromatique choisi parmi les radicaux suivants: thiényle-2, thiényle-3, naphtyle-1, naphtyle-2, (méthoxy-2 naphtyle)-1, méthylènedioxy-3,4 phényle, les phényles de formule générale (II) 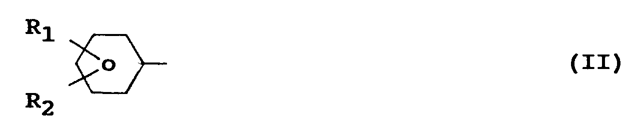 dans laquelle R₁ représente un atome d'hydrogène ou un radical hydroxyle ou alcoxyle en C₁-C₄ et R₂ représente un atome d'hydrogène ou un radical alcoyle en C₁-C₄, alcoxyle en C₁-C₄ ou hydroxyle ainsi que de ses sels de métal alcalin, caractérisé par le fait que l'on fait réagir dans un solvant , l'acide arylglycolique correspondant de formule générale (III) ou l'un des ses sels de métal alcalin
**Ar - CR(OH) - COOH (III)**
avec un agent donneur d'hydrogène choisi dans le groupe constitué par : l'acide formique, ou l'un de ses sels de métal alcalin, l'acide phosphonique, l'acide phosphinique ou l'un de leurs sels de métal alcalin, en présence d'un catalyseur de transfert d'hydrogène, que l'on isole ou, si désiré, l'on salifie.

2. Procédé selon la revendication 1, caractérisé par le fait que le solvant utilisé est un mélange d'eau et d'acide acétique.

3. Procédé selon la revendication 1, caractérisé par le fait que le solvant utilisé est l'eau.

4. Procédé selon l'une quelconque des revendications 1 à 3, caractérisé par le fait que le catalyseur de transfert d'hydrogène est du palladium.

5. Procédé selon l'une quelconque des revendications 1 à 4, caractérisé par le fait que l'agent donneur d'hydrogène est de l'acide formique ou l'un de ses sels de métal alcalin.

6. Procédé selon l'une quelconque des revendications 1 à 4, caractérisé par le fait que l'agent donneur d'hydrogène est de l'acide phosphonique ou l'un de ses sels de métal alcalin.

7. Procédé selon l'une quelconque des revendications 1 à 4, caractérisé par le fait que l'agent donneur d'hydrogène est de l'acide phosphinique ou l'un de ses sels de métal alcalin.

8. Procédé selon l'une quelconque des revendications 1 à 7, caractérisé par le fait qu'il est réalisé en présence de quantités catalytiques d'ions chlorure.

9. Procédé selon la revendication 8, caractérisé par le fait qu'il est réalisé en présence de 0,1 à 0,01 mole de chlorure de sodium par mole d'acide arylglycolique mis en oeuvre.

10. Application du procédé selon l'une quelconque des revendications 1 à 9 à l'obtention de l'acide parahydroxyphénylacétique ou de l'un de ses sels métaux alcalins.

11. Application du procédé selon l'une quelconque des revendications 1 à 9 à l'obtention de l'acide paraméthoxyphénylacétique ou de l'un de ses sels métaux alcalins.

12. Application du procédé selon l'une quelconque des revendications 1 à 9 à l'obtention de l'acide orthohydroxyphénylacétique ou de l'un de ses sels métaux alcalins.
